# EUROPÄISCHE PATENTSCHRIFT

(11) **EP 1 691 870 B1**
(45) Veröffentlichungstag und Bekanntmachung des Hinweises auf die Patenterteilung: **01.02.2012**
(21) Anmeldenummer: 04797647.7
(22) Anmeldetag: 05.11.2004
(51) Int. Cl.: A61M 15/00, A61K 9/00

(54) **PULVERINHALATOR**
POWDER INHALER
INHALATEUR DE POUDRE

(30) Priorität: 08.11.2003 DE 10352277
(43) Veröffentlichungstag der Anmeldung: 23.08.2006
(60) Teilanmeldung: 11172684.0
(73) Patentinhaber: Boehringer Ingelheim International GmbH, 55216 Ingelheim am Rhein (DE); Boehringer Ingelheim Pharma GmbH & Co.KG, 55216 Ingelheim am Rhein (DE)
(72) Erfinder: WACHTEL, Herbert, 55218 Ingelheim (DE)
(74) Vertreter: Hammann, Heinz
(86) Internationale Anmeldenummer: PCT/EP2004/012535
(87) Internationale Veröffentlichungsnummer: WO 2005/044353

(56) Entgegenhaltungen:
- WO-A-02/098874
- WO-A-03/084502
- US-A- 5 685 294

## Beschreibung

Die Erfindung betrifft einen Inhalator für die Inhalation pulverförmigerArzneimittel aus Kapseln, welche in eine in dem Inhalator angeordnete Kapselhalterung vor der Benutzung eingesteckt werden. Nach dem Einlegen der Kapsel in die Kapselhalterung kann der Patient ein Betätigungsorgan drücken, welches aus einer Ruhestellung in eine Bewegung versetzbar ist und dabei mit mindestens einer in die Kapselhalterung einstoßbaren Nadel zusammenwirkt. Mit Hilfe der mindestens einen Nadel wird die Kapsel angestochen und das Arzneimittel freigesetzt.

Ein derartiger Inhalator wird beispielsweise in der EP 0 703 800 B1 oder EP 0 911 047 A1 oder WO03/084502 A1 beschrieben. Der aus den vorstehend genannten Druckschriften bekannte Inhalator weist ein schalenförmiges Unterteil und einen hierzu passenden ebenfalls schalenartigen Deckel auf, welche über ein im Randbereich angeordnetes Gelenk zur Benutzung auseinander geklappt werden können. Zwischen dem Unterteil und dem Deckel greifen in dem Gelenk noch ein ebenfalls wegklappbares Mundstück und eine darunter befindliche Platte mit darunter angeordneter Kapselhalterung an. Nach dem Auseinanderklappen der einzelnen Baugruppen kann der Patient eine mit Arzneimittel gefüllte Kapsel in die Kapselhalterung einstecken, die Platte mit Kapselhalterung sowie das Mundstück in das Unterteil zurückschwenken und die Kapsel über ein seitlich aus dem Unterteil herausragendes federvorgespanntes Betätigungsorgan anstechen. Durch ein Saugen an dem Mundstück gelangt dann das Arzneimittel in die Atemwege des zu behandeinden Patienten. Das Dokument WO-A1-03/084502 offenbart einen Inhalator gemäß dem Oberbegriff des Anspruchs 1.

Im Hinblick auf die Handhabung sollen die bekannten Inhalatoren noch weiter verbessert werden.

Die Aufgabe wird erfindungsgemäß mit einem Inhalator gelöst, bei dem das Betätigungsorgan als Multifunktions-Betätigungsorgan ausgebildet ist, mittels dessen in einer ersten Funktionestellung das Verschlußelement zum Verschwenken des Deckels von dem Unterteil entrastbar ist, und mit welchem in einer zweiten Funktionsstellung das mit der Platte verrastete Mundstück von der Platte derart lösbar ist, dass das Mundstück von dem Unterteil wegschwenkbar ist.

Der wesentliche Vorteil der Erfindung liegt darin, dass die Kräfte zum Lösen des Deckels und Mundstückes aus der mechanischen Verrastung nicht direkt über den Deckel bzw. das Mundstück eingeleitet werden, sondern über das Multifunktions-Betätigungsorgan. Dadurch wird ein schnelles und sicheres Öffnen des Deckels und Mundstücks gewährleistet, um den Inhalator einsatzbereit zu machen. Dieses ist insbesondere bei einem beginnenden Asthmaanfall von großer Wichtigkeit. Die eigentliche Öffnungsbewegung des Deckels kann dann wie bisher durch Angreifen an den Deckel durch den Patienten vorgenommen werden.

Da die Kräfte zum Lösen der Bauteile Deckel und Mundstück deutlich niedriger sind, kann auf das Anbringen von Griffhilfen verzichtet werden. Unter Griffhilfen werden beispielsweise Griffmulden oder Riffelungen verstanden, die jedoch den Nachteil einer erhöhten Schmutzanfälligkeit haben können. Durch den Verzicht auf derartige Griffhilfen werden neben einem verbesserten optischen Erscheinungsbild auch die hygienischen Verhältnisse optimiert. Dieses ist besonders im Bereich des Mundstückes wichtig, da dieses Bauteil bei Benutzung des Inhalators in den Mundraum eingeführt wird.

In einer bevorzugten Ausführungsform können zur Unterstützung der Öffnungsbewegung noch Federelemente zwischen Deckel und Unterteil bzw. Mundstück und Unterteil angeordnet sein, die bei einer entsprechenden Dimensionierung ein schnappartiges Aufspringen des Deckels und/oder Mundstückes ermöglichen.

Vorzugsweise ist das Multifunktions-Betätigungsorgan verschiebbar an der Platte oder der Kapselhalterung gelagert. Die Platte und/oder die Kapselhalterung bildet bzw. bilden somit ein Widerlager für das Multifunktions-Betätigungsorgan, welches bei einem Verschieben aus der Ruhestellung in die jeweilige Funktionsstellung an der Platte entlanggleitet und durch diese beispielsweise über eine Führungsschiene geführt ist.

In einer günstigen Ausgestaltung ist das Multifunktions-Betätigungsorgan federvorgespannt. Aufgrund der bereits in der Ruhestellung auftretenden Rückstellkraft ist sichergestellt, dass nach der Benutzung des Inhalators das Multifunktions-Betätigungsorgan in die Ruhestellung zurückgeführt wird und somit für den nächsten Beschickungsvorgang mit einer neuen Kapsel und Inhalationsvorgang bereit ist.

Vorteilhafterweise weist das Multifunktions-Betätigungsorgan einen Hauptkörper und zwei daran angreifende parallele Führungsarme auf. Dabei ragen die Führungsarme in das Unterteil hinein und dienen mit entsprechenden Einbauteilen, beispielsweise mit außenseitig an der Kapselhalterung angeordneten Führungshülsen, der Führung des Multifunktions-Betätigungsorgans während der Bewegung aus der Ruhestellung in die jeweiligen Funktionsstellungen und zurück in die Ruhestellung.

Die Führungsarme können an ihrem hauptkörperfernen Ende Endanschläge aufweisen, welche in der Ruhestellung an den Führungshülsen anliegen. Dadurch wird eine Federspannung des Multifunktions-Betätigungsorgans aufgebaut.

In einer bevorzugten Ausgestaltung weist der Hauptkörper eine obere und eine seitliche Riffelfläche auf. Diese liegen aber im Gegensatz zum Stand der Technik nicht an dem Deckel und insbesondere nicht an dem Mundstück, sondern ausschließlich an dem Hauptkörper des Multifunktions-Betätigungsorgans außerhalb des Inhalators und kommen demzufolge nicht in den Mundbereich des Patienten. Darüber hinaus können die Riffelflächen flächenmäßig kleiner ausgeführt sein und bieten dennoch eine Gewähr für eine sichere und schnelle Benutzung des Inhalators.

Günstigerweise ist die obere Riffelfläche in der Ruhestellung in ihrem deckelseitigen Bereich mit einer Ausnehmung zur Aufnahme des Verschlusselementes des Deckels ausgebildet. Innerhalb der Ausnehmung kann mindestens die in Richtung der seitlichen Riffelfläche gerichtete Seitenwand derart geneigt sein, dass sie beim Einschieben des Hauptkörpers eine Gleitfläche für das Verschlusselement bildet und dadurch das Verschlusselement zusammen mit dem Deckel aus der verrasteten Stellung gehoben wird.

In der bisher beschriebenen ersten besonders günstigen Ausführungsform zum Lösen des Mundstückes umfasst das Multifunktions-Betätigungsorgan einen an dem Hauptkörper angreifenden Schubfortsatz und eine an dem Mundstück angeordnete, die Platte hintergreifende Haltenase, wobei der Schubfortsatz derart ausgerichtet ist, dass er bei Erreichen der zweiten Funktionsstellung mit seiner Vorderkante auf die Haltenase trifft. Bei weiterem Vorschieben des Hauptkörpers drückt der Schubfortsatz die vorzugsweise biegeweiche Haltenase weiter zurück, bis diese aus der Öffnung der ortsfesten Platte rutscht und das Mundstück freigibt.

In einer zweiten bevorzugten Ausführungsform zum Lösen des Mundstückes umfasst das Multifunktions-Betätigungsorgan einen an einem Hauptkörper verschwenkbar gelagerten Schwenkkörper, an welchem ein in den Inhalator hineinragender Stoßnocken angeordnet ist, und einen mit dem Stoßnocken zusammenwirkenden an dem Unterteil angebrachten Haltearm, welcher in der Verschlussposition durch die Platte hindurch das Mundstück hintergreift, wobei der Stoßnocken in der Ruhestellung seitlich an dem Haltearm vorbeiragt. Vorzugsweise ist dabei der Stoßnocken durch eine Öffnung in dem Hauptkörper hindurchgeführt. Zum Öffnen des Mundstückes schwenkt der Patient den Schwenkkörper und somit auch den Stoßnocken, welcher dem Abschnitt einer Kreisbahn folgend auf der Innenseite des Inhalators gegen den mit dem Mundstück verbundenen Haltearm trifft und bei Aufbringen weiteren Druckes diesen verbiegt. Hierdurch rutscht der an seinem oberen Ende vorzugsweise mit einem Haltehaken versehene Haltearm aus dem Mundstück und gibt dieses frei.

In einer vorteilhaften Ausgestaltung ist der Stoßnocken exzentrisch an dem Schwenkkörper angeordnet. Diese Anordnung ermöglicht in Abhängigkeit der Anordnung des Haltearms eine möglichst direkte Kraftübertragung auf diesen.

Alternativ zu den vorstehend beschriebenen Ausführungsformen zum Lösen des Mundstückes kann im Unterteil mindestens ein Betätigungsfenster mit daran ausgebildeten Funktionselementen angeordnet sein, wobei das mindestens eine Betätigungsfenster federvorgespannt und geführt in das Unterteil drückbar ist und über die Funktionselemente das mit der Platte verrastete Mundstück von der Platte löst. Bei dieser Ausführungsform übernimmt die Baugruppe um das Betätigungsorgan das Lösen des Deckels, während der als Betätigungsfenster ausgebildete Druckknopf im Unterteil zusammen mit weiteren Komponenten das Lösen des Mundstückes durch den Patienten auslöst. Die Betätigungsfenster erlauben dem Patienten die Kontrolle, ob eine Kapsel mit dem Arzneimittel in die Kapselhalterung eingesetzt ist. Bei dieser Alternativ handelt es sich nicht um eine Ausführungsform der Erfindung, sondern um ein Beispiel eines Inhalators mit eindrückbarem Betätigungsfenster im Unterteil zum lösen des Mundstückes.

Vorzugsweise ist jeweils ein Betätigungsfenster gegenüberliegend in dem Unterteil angeordnet ist. Sofern diese miteinander spangenartig verbunden sind, ergibt sich bei entsprechender Materialwahl eine materialimmanente Rückstellkraft, so dass auf weitere Federelemente verzichtet werden kann.

Günstigerweise umfassen die Funktionselemente Haken und komplementär dazu ausgebildete Halteelemente, wobei die Haken einstückig mit dem mindestens einen Fenster ausgebildet sein und sich stegartig in Richtung des Mundstückes erstrecken können. In dem Mundstück wiederum befinden sich die Halteelemente, beispielsweise Ösen oder Haltekragen, in welche die Haken eingreifen können und das Mundstück dadurch verrasten. Bei einem Eindrücken des mindestens einen Betätigungsfensters weicht auch der Haken in den Innenraum des Unterteils zurück, so dass der Haken aus dem Halteelement rutscht und das Mundstück weggeklappt werden kann.

Vorteilhafterweise ist die mit dem Unterteil verrastete Platte von dem Unterteil derart lösbar, dass die Platte von dem Unterteil wegschwenkbar ist. Die Verrastung zwischen Platte und Unterteil kann mittels vorstehender Haltelaschen realisiert werden.

Auch ist es möglich, den Inhalator gemäß allen Ausführungsformen derart auszuführen, dass das Betätigungsorgan mit der mindestens einen in die Kapselhalterung einstoßbaren Nadel so mit der Platte verbunden ist, dass es zusammen mit der mit dem Unterteil verrasteten Platte vom Unterteil gelöst und weggeschwenkt werden kann.

Zum besseren Verständnis der Erfindung, wird diese nunmehr anhand der nachfolgenden drei Zeichnungsfiguren näher erläutert. Dabei zeigen die:
- **Fig.1:**: eine Explosionsdarstellung mit Multifunktions-Betätigungsorgan in einer Ausführungsform mit Hauptkörper und daran angeordnetem Schubfortsatz;
- **Fig. 1a:**: einen vergrößerten Ausschnitt von Platte, Kapselhalterung und Multifunktions-Betätigungsorgan gemäß Fig. 1;
- **Fig. 2:**: eine Explosionsdarstellung einer Ausführungsform mit eindrückbaren Betätigungsfenstern im Unterteil; Bei der Figur 2 handelt es sich nicht um eine Ausführungsform der Erfindung, sondern um ein Beispiel eines Inhalators mit eindrückbaren Betätigunsfenstern im Unterteil zum Lösen des Mundstückes.
- **Fig. 3:**: eine Explosionsdarstellung mit Multifunktions-Betätigungsorgan in einer Ausführungsform mit Hauptkörper und Schwenkkörper.

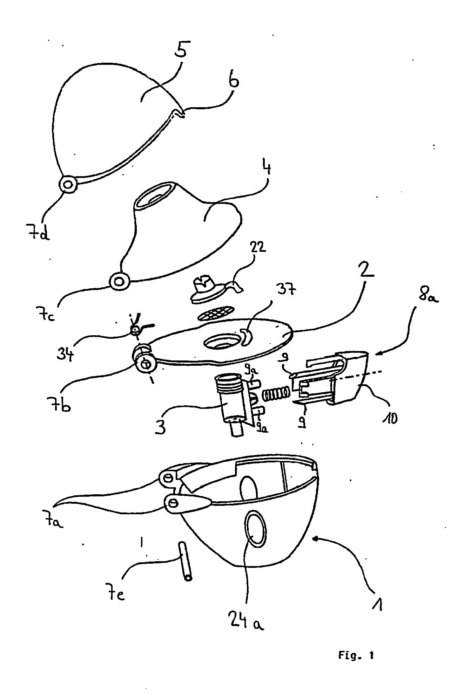

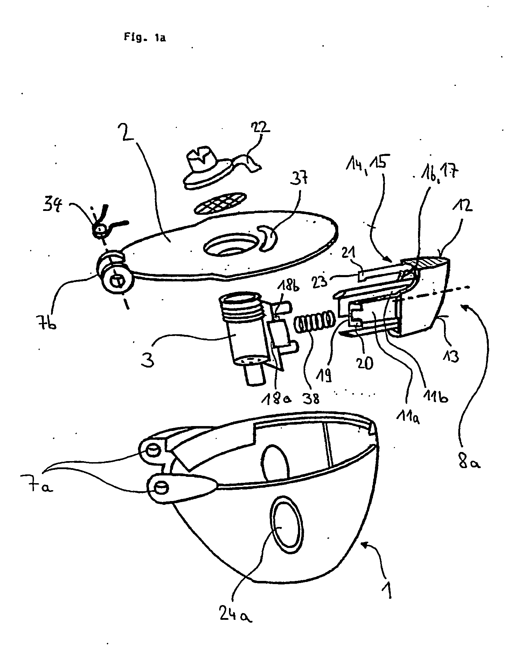

In der Fig. 1 ist der Inhalator in einer Explosionsdarstellung gezeigt Die wesentlichen Baugruppen sind dabei das Unterteil 1, welches die Platte 2 aufnimmt und durch diese abgedeckt wird, das an dem Unterteil 1 über die Haltenase 22 verrastbare Mundstück 4 und den komplementär zu dem Unterteil 1 ausgebildeten Deckel 5.

In geschlossenem Zustand des Inhalators greift das Verschlusselement 6 an die Platte 2 und wird dort kraftschlüssig gehalten. Auch ist es möglich durch wulstartige Ausbildungen einen Formschluss zu realisieren. Für ein Angreifen des Verschlusselementes 6 an die Platte 2 weist der Hauptkörper 10 des Multifunktions-Betätigungsorgans 8a eine Ausnehmung 15 auf, wie in der vergrößerten Ansicht der Fig. 1 a am besten zu erkennen ist. Die Ausnehmung 15 ist mit einer geneigten Seitenwand 16 versehen und befindet sich in dem deckelseitigen Bereich 14 der oberen Riffelfläche 12. Für eine besonders sichere Bedienung ist das Multifunktions-Betätigungsorgan 8a außerdem mit einer seitlichen Riffelfläche 13 versehen.

Zum Öffnen des Deckels 5 wird zunächst das Multifunktions-Betätigungsorgan 8a in eine erste Funktionsstellung vorgeschoben. Dabei stößt das Verschlusselement 6 auf die geneigte Seitenwand 16, welche bei weiterem Vorschub des Hauptkörpers 10 als Gleitfläche 17 wirkt und für ein Lösen des Deckels 5 sorgt.

Das Unterteil 1 ist becherartig ausgeformt und nimmt vollständig die an der Unterseite der Platte 2 angeordnete Kapselhalterung 3 auf. Um eine mit Arzneimittel gefüllte Kapsel (nicht dargestellt) einlegen zu können muss jedoch auch noch das Mundstück 4 weggeklappt werden. In der Ausführungsform gemäß der Fig. 1 erfolgt dieses durch weiteres Hineindrücken des Multifunktions-Betätigungsorgans 8a in eine zweite Funktionsstellung. Dabei stößt ein an dem Hauptkörper 10 angebrachter und in Bewegungsrichtung des Hauptteils 10 ausgerichteter Schubfortsatz 21 mit seiner Vorderkante 23 (Fig. 1 a) unterhalb der Platte 2 auf eine durch die Platte 2 hindurchragende, ortsfest an dem Mundstück 4 angebrachte Haltenase 22. Die Platte 2 weist im Bereich der Durchführung der Haltenase 22 eine Haltenasenöffnung 37 auf.

Durch die Vorschubbewegung des Schubfortsatzes 21 verbiegt sich die Haltenase 22 und entweicht durch die Haltenasenöffnung 37, wodurch das Mundstück 4 wegschwenkt. Das Wegschwenken wird durch eine Sprungfeder 34 unterstützt.

In dieser geöffneten Position von Deckel 5 und Mundstück 4 kann die Kapsel in die Kapselhalterung 3 eingelegt werden. Zum Freisetzen des Wirkstoffes sind an dem Hauptkörper zwei senkrecht versetzte und parallel verlaufende Nadeln 9 angebracht, die sich kontinuierlich mit dem Einschieben des Hauptkörpers 10 in Richtung der Kapsel (nicht dargestellt) bewegen und diese perforieren. Der Perforiervorgang kann durch ein Sichtfenster 24a beobachtet werden. Nachfolgend wird das Mundstück 4 wieder zurückgeschwenkt.

In der Kapselhalterung 3 sind rohrförmige Nadeldurchführungen 9a zu erkennen, die axial entsprechend der Bewegungsrichtung der Nadeln 9 ausgerichtet sind. Diese sorgen einerseits für ein treffsicheres Aufsetzen der Nadeln auf der Kapsel (nicht dargestellt) und andererseits für eine zusätzliche Führung des Multifunktions-Betätigungsorgans 8a. Die wesentliche Führung wird jedoch über zwei seitlich an dem Hauptkörper 10 angeordnete Führungsarme 11 a, 11 b realisiert. Die Führungsarme 11 a, 11 b haben darüber hinaus die Aufgabe das Multifunktions-Betätigungsorgan 8a unter einer Vorspannung zu halten. Hierfür sind die Führungsarme 11 a, 11 b an ihrem hauptkörperfemen Ende 19 mit Endanschlägen 20 versehen, die in der Ruhestellung des Multifunktions-Betätigungsorgans 8a an den Führungshülsen 18a, 18b anliegen. Die Führungshülsen 18a, 18b befinden sich an der Außenseite der Kapselhalterung 3. Zwischen den Nadeln 9 ist eine Schraubenfeder 38 angeordnet, die in ihrer axialen Erstreckung parallel zu den Nadeln 9 verläuft, wobei die Schraubenfeder 38 derart mit der Länge der Führungsarme 11 a, 11 b abgestimmt ist, dass das Multifunktions-Betätigungsorgan 8a auch in der Ruhestellung noch vorgespannt ist.

Die einzelnen Baugruppen Unterteil 1, Platte 2, Mundstück 4 und Deckel 5 werden über Gelenkaufnahmen 7a, 7b, 7c 7d und einen Gelenkbolzen 7e verbunden.

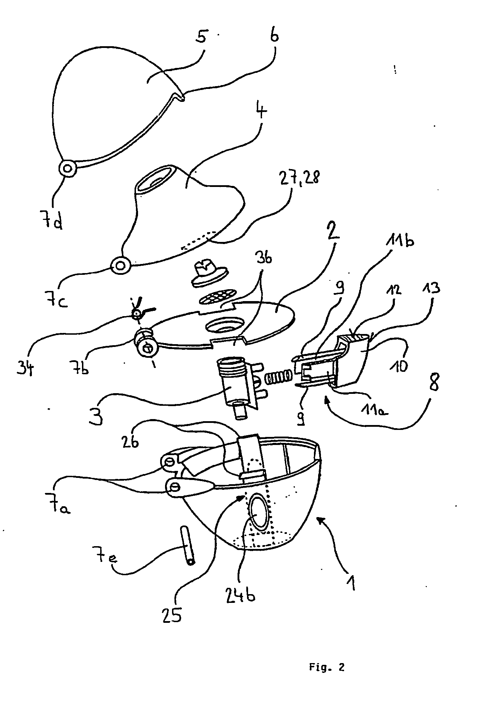

Die Fig. 2 zeigt eine Darstellung ähnlich der Fig. 1, bei der das Öffnen des Deckels 5 mit dem Erreichen der ersten Funktionsstellung durch Betätigung des Hauptkörpers 10 des Betätigungsorgans 8 erfolgt, vergleichbar mit der in der Fig. 1 dargestellten Ausführungsform.

Nach dem Öffnen des Deckels 5 ist es notwendig, ebenfalls das Mundstück 4 wegzuklappen, um in die Kapselhalterung 3 eine mit Arzneimittel gefüllte Kapsel (nicht dargestellt) einfügen zu können. Hierzu drückt der Patient die beidseitig an dem Unterteil 1 angebrachten Betätigungsfenster 24 b. Durch den Druckvorgang auf das Betätigungsfenster 24b biegen sich die Funktionselemente 25, die in der Fig. 2 als mit dem Fenster integrierte aufrechte Stege ausgebildet sind, nach innen in den Hohlraum des Unterteils 1. Am Ende der Funktionselemente sind Haken 26 ausgeformt, die über das Unterteil 1 hinausragen und bei geschlossener Platte 2 es ermöglichen, bei auf der Platte 1 aufgesetztem Mundstück 4 in die auf der Innenseite des Mundstückes 4 befindliche Halteelemente 27 einzugreifen. Für den Fall des wegzuklappenden Mundstückes 4 rutschen bei Aufbringen einer Druckkraft auf das Betätigungsfenster 24b die Haken 26 aus den Halteelementen 27, die wie im dargestellten Fall aus einem Haltekragen 28 bestehen.

Nach Einsetzen der Kapsel (nicht dargestellt) in die Kapselhalterung 3 wird das Mundstück 4 zurückgeschwenkt, bis es mit den durch die Hakendurchführung 36 herausstehenden Haken 26 verrastet. Nachfolgend kann nunmehr durch Drücken des Hauptkörpers 10 die Kapsel (nicht dargestellt) angestochen werden. Hierfür stehen in Richtung der Kapselhalterung 3 zwei parallele an dem Hauptkörper 10 befestigte Nadeln 9 hervor. Nach der Benutzung wird der Deckel 5 wieder verschlossen. Hierbei rastet das Verschlusselement 6 an der Platte 2 ein.

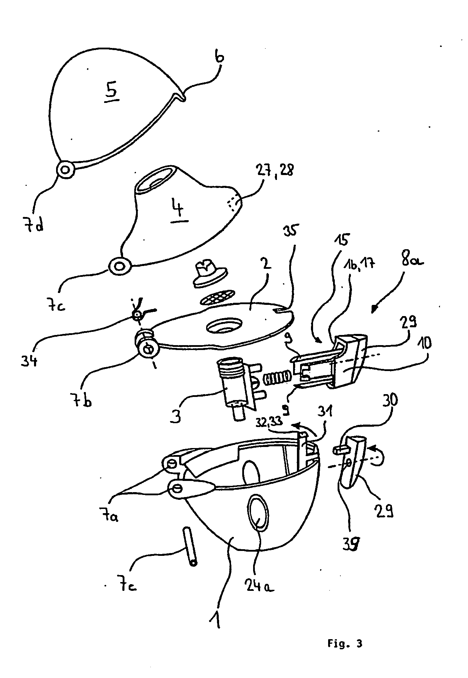

Die Fig. 3 zeigt eine weitere Ausführungsform, bei der ein Wegklappen des Mundstückes 4 auf einem anderen konstruktiven Weg erreicht wird. Hierzu ist das Multifunktions-Betätigungsorgan 8a zweiteilig ausgeführt. Neben dem zum Öffnen des Deckels 5 verschiebbar angeordneten Hauptteil 10 befindet sich an dessen äußeren Seite, verdrehbar zu dem Hauptteil 10, der Schwenkkörper 29. Der Schwenkkörper 29 ist zentrisch an dem Hauptteil 10 auf einem Zapfen befestigt. Oberhalb der Lagerbohrung 39 ist exzentrisch der Stoßnocken 30 angeordnet, der bei zusammengesetztem Inhalator in den Innenraum des Unterteils 1 hineinragt und bei einer Drehbewegung in der in Fig. 3 dargestellten Pfeilrichtung auf den aufrecht emporstehenden Haltearm 31 auftrifft. Hierdurch wird wiederum das obere Ende 32 des Haltearms 31 ausgelenkt und der daran befindliche Haltehaken 33 rutscht aus dem im Mundstück 4 befindlichen Halteelement 27, welches ebenfalls als Haltekragen 28 ausgeformt ist. Damit ein störungsfreies Herausrutschen des Haltehakens 33 aus dem Haltekragen 28 möglich ist, weist die Platte 2 eine ausreichend groß dimensionierte Haltearmdurchführung 35 auf.

Zum Schließen des Mundstückes 4 wird dieser in die ursprüngliche Position zurückgeschwenkt. Durch die in Richtung des Deckels 5 spitzen Formgebung des Haltehakens 33 rutscht dieser durch die Haltearmdurchführung 35 auf die Innenseite des Mundstückes 4 und hintergreift dort aufgrund der in den Haltehaken 33 vorhandenen Rückstellkräfte die ausgeformten Haltekragen 28.

### Bezugszeichenliste

- 1: Unterteil
- 2: Platte
- 3: Kapselhalterung
- 4: Mundstück
- 5: Deckel
- 6: Verschlusselement
- 7a, 7b, 7c, 7d: Gelenkaufnahmen
- 7e: Gelenkbolzen
- 8: Betätigungsorgan
- 8a: Multifunktions-Betätigungsorgan
- 9: Nadel
- 9a: Nadeldurchführungen
- 10: Hauptkörper
- 11 a, 11 b: Führungsarm
- 12: obere Riffelfläche
- 13: seitliche Riffelfläche
- 14: deckelseitiger Bereich
- 15: Ausnehmung
- 16: Seitenwand der Ausnehmung
- 17: Gleitfläche
- 18a, 18b: Führungshülse
- 19: hauptkörperfernes Ende der Führungsarme
- 20: Endanschläge
- 21: Schubfortsatz
- 22: Haltenase
- 23: Vorderkante Schubfortsatz
- 24a: Sichtfenster
- 24b: Betätigungsfenster
- 25: Funktionselemente
- 26: Haken
- 27: Halteelemente
- 28: Haltekragen
- 29: Schwenkkörper
- 30: Stoßnocken
- 31: Haltearm
- 32: Oberes Ende Haltearm
- 33: Haltehaken
- 34: Sprungfeder (Gehäuse)
- 35: Haltearmdurchführung
- 36: Hakendurchführung
- 37: Haltenasenöffnung
- 38: Schraubenfeder
- 39: Lagerbohrung

## Patentansprüche

1. Inhalatorfür die Inhalation pulverförmiger Arzneimittel aus Kapseln, aufweisend
- ein Unterteil (1)
- ein mit dem Unterteil (1) verrastbare Platte (2), mit welcher das Unterteil (1) verschließbar ist, und eine in dem Unterteil (1) versenkbare Kapselhalterung (3) zur Aufnahme der Kapseln,
- ein mit der Platte (2) verrastbares Mundstück (4),
- einen Deckel (5), welcher das Mundstück (4) in einer Verschlussposition abdeckt und mittels eines Verschlusselementes (6) verrastet, wobei das Unterted (1), die Platte (2), das Mundstück (4) und der Deckel (5) durch ein einziges Gelenk (7) miteinander gelenkig verbunden sind, und
- ein Betätigungsorgan, welches aus einer Ruhestellung in eine Bewegung versetzbar ist und dabei mit mindestens einer in die Kapselhalterung (3) einstoßbaren Nadel (9) zusammenwirkt,
**dadurch gekennzeichnet, dass** das Betätigungsorgan als Multifunktions-Betätigungsorgan (8a) ausgebildet Ist, mittels dessen in einer ersten Funktionsstellung das Verschlusselement (6) zum Verschwenken des Deckels (5) von dem Unterteil (1) entrastbar ist, und mit welchem in einer zweiten Funktionsstellung das mit der Platte (2) verrastete Mundstück (4) von der Platte (2) derart lösbar ist, dass das Mundstück (4) von dem Unterteil (1) wegschwenkbar ist.

2. Inhalator nach Anspruch 1, **dadurch gekennzeichnet, dass** das Multifunktions-Betätigungsorgan (8a) verschiebbar an der Platte (2) und/oder der Kapselhalterung (3) gelagert ist.

3. Inhalator nach Anspruch 1 oder 2, **dadurch gekennzeichnet, dass** das Multifunktions-Betätigungsorgan (8a) federvorgespannt ist.

4. Inhalator nach einem der Ansprüche 1 bis 3, **dadurch gekennzeichnet, dass** das Multifunktions-Setätigungsorgan (8a) einen Hauptkörper (10) und zwei daran angreifende parallele Führungsarme (11a, 11b) aufweist.

5. Inhalator nach Anspruch 4, **dadurch gekennzeichnet, dass** das der Hauptkörper (10) eine obere und eine seitliche Riffelfläche (12, 13) ausweist.

6. Inhalator nach Anspruch 5, **dadurch gekennzeichnet, dass** die obere Riffelfläche (12) in der Ruhestellung in ihrem deckelseitigen Bereich (14) mit einer Ausnehmung (15) zur Aufnahme des Verschlusselementes (6) ausgebildet ist.

7. Inhalator nach Anspruch 6, **dadurch gekennzeichnet, dass** mindestens die in Richtung der seitlichen Riffelfläche (13) gerichtete Seitenwand (16) der Ausnehmung (15) derart geneigt ist, dass sie eine Gleitfläche (17) für das Verschlusselement (6) bildet.

8. Inhalator nach einem der Ansprüche 4 bis 7, **dadurch gekennzeichnet, dass** die Führungsarme (11a, 11b) durch außenseitig an der Kapselhalterung (3) angeordnete Führungshülsen (18a, 18b) geführt sind.

9. Inhalator nach Anspruch 8, **dadurch gekennzeichnet, dass** die Führungsarme (11a, 11 b) an ihrem hauptkörperfernenn Ende (19) Endanschläge (20) aufweisen, welche in der Ruhestellung an den Führungshülsen (18a, 18b) anliegen.

10. Inhalator nach Anspruch 1, **dadurch gekennzeichnet, dass** das Multifunktions-Betätigungsorgan (8a) einen Hauptkörper (10) aufweist und das Multifunktions-Betätigungsorgan (8a) einen an dem Hauptkörper (10) angreifenden Schubfortsatz (21) und eine an dem Mundstück (4) angeordnete, die Platte (2) hintergreifende Haltenase (22) umfasst, wobei der Schubfortsatz (21) derart ausgerichtet ist, dass er bei Erreichen der zweiten Funktionsstellung mit seiner Vorderkante (23) auf die Haltenase (22) trifft.

11. Inhalator nach Anspruch 1, **dadurch gekennzeichnet, dass** das Multifunktions-Betätigungsorgan (8a) einen an einem Hauptkörper (10) verschwenkbar gelagerten Schwenkkörper (29) umfasst, an welchem ein in den Inhalator hineinragender Stoßnocken (30) angeordnet ist, und einen mit dem Stoßnocken (30) zusammenwirkenden an dem Unterteil angebrachten Haltearm (31), welcher in der Verschlussposition durch die Platte (2) hindurch das Mundstück (4) hintergreift, wobei der Stoßnocken (30) in der Ruhestellung seitlich an dem Haltearm (31) vorbeiragt.

12. Inhalator nach Anspruch 11, **dadurch gekennzeichnet, dass** der Stoßnocken (30) exzentrisch an dem Schwenkkörper (29) angeordnet ist.

13. Inhalator nach Anspruch 11 oder 12, **dadurch gekennzeichnet, dass** der Haltearm (31) an seinem oberen Ende (32) einen Haltehaken (33) aufweist.

14. Inhalator nach einem der Ansprüche 1 bis 13, **dadurch gekennzeichnet, dass** die mit dem Unterteil (1) verrastete Platte (2) vom Unterteils (1) derart lösbar ist, dass die Platte (2) von dem Unterteil (1) wegschwenkbar ist.

15. Inhalator nach einem der Ansprüche 1 bis 14, **dadurch gekennzeichnet dass** das Betätigungsorgan so mit der Platte (2) verbunden ist, dass es zusammen mit der mit dem Unterteil (1) verrasteten Platte (2) vom Unterteil (1) gelöst und weggeschwenkt werden kann.

## Claims

1. Inhaler for inhaling powdered medicament from capsules, comprising
- a lower part (1)
- a plate (2) capable of latching to the lower part (1), with which the lower part (1) can be closed, and a capsule holder (3) for receiving the capsules wherein the capsule holder (3) can be countersunk in the lower part (1),
- a mouthpiece (4) capable of latching to the plate (2),
- a cover (5) which covers the mouthpiece (4) in a closed position and latches by means of a closure element (6), the lower part (1), the plate (2), the mouthpiece (4) and the cover (5) being jointed to one another by a single joint (7), and
- an actuating member (8) which can be set in motion from a resting position and thereby co-operates with at least one pin (9) that can be pushed into the capsule holder (3),
**characterised in that** the actuating member (8) is constructed as a multi-functional actuating member (8a) by means of which, in a first functional position, the closure element (6) can be disengaged in order to pivot the cover (5) from the lower part (1), and with which in a second functional position the mouthpiece (4) engaging with the plate (2) can be released from the plate (2) such that the mouthpiece (4) can be pivoted away from the lower part (1).

2. Inhaler according to claim 1, **characterised in that** the multi-functional actuating member (8a) is movably mounted on the plate (2) and/or the capsule holder (3).

3. Inhaler according to claim 1 or 2, **characterised in that** the multi-functional actuating member (8a) is spring-loaded.

4. Inhaler according to one of claims 1 to 3, **characterised in that** the multi-functional actuating member (8a) comprises a main body (10) and two parallel guide arms (11 a, 11 b) acting thereon.

5. Inhaler according to claim 4, **characterised in that** the main body (10) comprises an upper and a lateral grooved surface (12, 13).

6. Inhaler according to claim 5, **characterised in that** the upper grooved surface (12) in the resting position has a recess (15) in its region close to the cover (14) for accommodating the closure element (6).

7. Inhaler according to claim 6, **characterised in that** the recess (15) has a side wall and that at least the side wall (16) directed towards the lateral grooved surface (13) is inclined so that it forms a sliding surface (17) for the closure element (6).

8. Inhaler according to one of claims 4 to 7, **characterised in that** the guide arms (11a, 11b) are guided by guide sleeves (18a, 18b) provided on the outside of the capsule holder (3).

9. Inhaler according to claim 8, **characterised in that** the guide arms (11a, 11 b) comprise, at their end (19) remote from the main body, end stops (20) which abut on the guide sleeves (18a, 18b) in the resting position.

10. Inhaler according to claim 1, **characterised in that** the multi-functional actuating member (8a) comprises a main body (10) and the multi-functional actuating member (8a) comprises a thrust projection (21) acting on the main body (10) and a retaining lug (22) mounted on the mouthpiece (4) and engaging behind the plate (2), the thrust projection (21) being aligned so that on reaching the second functional position its front edge (23) makes contact with the retaining lug (22).

11. Inhaler according to claim 1, **characterised in that** the multi-functional actuating member (8a) comprises a pivot member (29) pivotably mounted on a main body (10), wherein an impact cam (30) is disposed on the pivot member (29) and is projecting into the inhaler, and a retaining arm (31) cooperating with the impact cam (30) and mounted on the lower part, wherein in the closed position the retaining arm (31) engages behind the mouthpiece (4) through the plate (2), the impact cam (30) projecting laterally past the retaining arm (31) in the resting position.

12. Inhaler according to claim 11, **characterised in that** the impact cam (30) is eccentrically mounted on the pivot member (29).

13. Inhaler according to claim 11 or 12, **characterised in that** the retaining arm (31) comprises a retaining hook (33) on its upper end (32).

14. Inhaler according to one of claims 1 to 13, **characterised in that** the plate (2) engaging with the lower part (1) can be released from the lower part (1) such that the plate (2) can be pivoted away from the lower part (1).

15. Inhaler according to one of claims 1 to 14, **characterised in that** the actuating member (8) is attached to the plate (2) such that it can be released from the lower part (1) and pivoted away together with the plate (2) latched to the lower part (1).

## Revendications

1. Inhalateur pour l'inhalation de médicaments sous forme de poudre se trouvant dans des capsules, comprenant
- une partie inférieure (1)
- une plaque (2), qui peut être encliquetée avec la partie inférieure (1) et par laquelle la partie inférieure (1) peut être fermée, et un support de capsule (3) servant à recevoir les capsules et pouvant s'enfoncer dans la partie inférieure (1),
- un embout buccal (4), qui peut être encliqueté avec la plaque (2),
- un couvercle (5), qui recouvre l'embout buccal (4) dans une position fermée et s'encliquette au moyen d'un élément de fermeture (6), la partie inférieure (1), la plaque (2), l'embout buccal (4) et le couvercle (5) étant reliés entre eux de manière articulée par une unique articulation (7), et
- un organe d'actionnement (8), qui peut être mis en mouvement à partir d'une position de repos et qui coopère alors avec au moins une aiguille (9) pouvant être poussée dans le support de capsule (3),
**caractérisé en ce que** l'organe d'actionnement (8) est réalisé sous forme d'organe d'actionnement polyvalent (8a) à l'aide duquel, dans une première position fonctionnelle, l'élément de fermeture (6) peut être désencliqueté de la partie inférieure (1) afin de faire pivoter le couvercle (5), et à l'aide duquel, dans une deuxième position fonctionnelle, l'embout buccal (4) encliqueté avec la plaque (2) peut être libéré de la plaque (2) de telle sorte que l'embout buccal (4) peut être éloigné de la partie inférieure (1) par pivotement.

2. Inhalateur selon la revendication 1, **caractérisé en ce que** l'organe d'actionnement polyvalent (8a) est monté à déplacement sur la plaque (2) et/ou sur le support de capsule (3).

3. Inhalateur selon la revendication 1 ou 2, **caractérisé en ce que** l'organe d'actionnement polyvalent (8a) est précontraint par ressort.

4. Inhalateur selon l'une quelconque des revendications 1 à 3, **caractérisé en ce que** l'organe d'actionnement polyvalent (8a) présente un corps principal (10) et deux bras de guidage parallèles (11a, 11b) qui agissent sur le corps principal.

5. Inhalateur selon la revendication 4, **caractérisé en ce que** le corps principal (10) présente une face supérieure et une face latérale cannelées (12, 13).

6. Inhalateur selon la revendication 5, **caractérisé en ce que** la face supérieure cannelée (12), dans la position de repos, possède dans sa région côté couvercle (14) un évidement (15) pour recevoir l'élément de fermeture (6).

7. Inhalateur selon la revendication 6, **caractérisé en ce qu'**au moins la paroi latérale (16) de l'évidement (15) qui est dirigée vers la face latérale cannelée (13) est inclinée de telle sorte qu'elle forme une surface de glissement (17) pour l'élément de fermeture (6).

8. Inhalateur selon l'une quelconque des revendications 4 à 7, **caractérisé en ce que** les bras de guidage (11a, 11b) sont guidés par des douilles de guidage (18a, 18b) disposées sur le côté extérieur du support de capsule (3).

9. Inhalateur selon la revendication 8, **caractérisé en ce que** les bras de guidage (11a, 11b) présentent, à leur extrémité (19) éloignée du corps principal, des butées terminales (20) qui s'appliquent dans la position de repos contre les douilles de guidage (18a, 18b).

10. Inhalateur selon la revendication 1, **caractérisé en ce que** l'organe d'actionnement polyvalent (8a) présente un corps principal (10) et l'organe d'actionnement polyvalent (8a) comprend un prolongement de poussée (21) agissant sur le corps principal (10) et un talon de retenue (22) disposé sur l'embout buccal (4) et s'engageant derrière la plaque (2), le prolongement de poussée (21) étant aligné de telle sorte qu'à l'atteinte de la deuxième position fonctionnelle, il rencontre par son bord avant (23) le talon de retenue (22).

11. Inhalateur selon la revendication 1, **caractérisé en ce que** l'organe d'actionnement polyvalent (8a) comprend un corps pivotant (29), monté à pivotement sur un corps principal (10) et sur lequel est disposé un ergot d'impact (30) s'enfonçant dans l'inhalateur, et un bras de retenue (31) coopérant avec l'ergot d'impact (30) et monté sur la partie inférieure, bras qui, dans la position fermée, s'engage derrière l'embout buccal (4) en traversant la plaque (2), sachant que, dans la position de repos, l'ergot d'impact (30) dépasse latéralement le bras de retenue (31).

12. Inhalateur selon la revendication 11, **caractérisé en ce que** l'ergot d'impact (30) est disposé excentriquement sur le corps pivotant (29).

13. Inhalateur selon la revendication 11 ou 12, **caractérisé en ce que** le bras de retenue (31) présente un crochet de retenue (33) à son extrémité supérieure (32).

14. Inhalateur selon l'une quelconque des revendications 1 à 13, **caractérisé en ce que** la plaque (2) encliquetée avec la partie inférieure (1) peut être libérée de la partie inférieure (1) de telle sorte que la plaque (2) peut être éloignée de la partie inférieure (1) par pivotement.

15. Inhalateur selon l'une quelconque des revendications 1 à 14, **caractérisé en ce que** l'organe d'actionnement (8) est relié à la plaque (2) de telle sorte qu'il peut être libéré et éloigné par pivotement de la partie inférieure (1) conjointement avec la plaque (2) encliquetée avec la partie inférieure (1).
